# EUROPEAN PATENT SPECIFICATION

(11) **EP 1 877 782 B1**
(45) Date of publication and mention of the grant of the patent: **05.10.2016**
(21) Application number: 06707618.2
(22) Date of filing: 21.03.2006
(51) Int. Cl.: G01N 33/50, G01N 33/68, G01N 33/543, C07K 16/28

(54) **METHOD FOR IDENTIFYING CELL-SPECIFIC PROTEIN COMBINATION PATTERNS**
VERFAHREN ZUR IDENTIFIZIERUNG ZELLSPEZIFISCHER PROTEINKOMBINATIONSMUSTER
PROCEDE PERMETTANT D'IDENTIFIER DES MOTIFS DE COMBINAISON DE PROTEINES SPECIFIQUES DE CELLULE

(30) Priority: 21.03.2005 US 663834 P
(43) Date of publication of application: 16.01.2008
(73) Proprietor: Schubert, Walter, 39175 Biederitz (DE)
(72) Inventor: SCHUBERT, Walter, 39175 Biederitz (DE)
(74) Representative: Hofstetter, Schurack & Partner
(86) International application number: PCT/EP2006/002569
(87) International publication number: WO 2006/100033

(56) References cited:
- EP-A- 0 810 428
- WO-A-01/36939
- US-A1- 2003 096 318
- US-A1- 2003 175 832
- US-B1- 6 638 506
- BELOV LARISSA ET AL: "Immunophenotyping of leukemias using a cluster of differentiation antibody microarray" CANCER RESEARCH, AMERICAN ASSOCIATION FOR CANCER RESEARCH, BALTIMORE, MD, US, vol. 61, no. 11, 1 June 2001 (2001-06-01), pages 4483-4489, XP002257440 ISSN: 0008-5472
- ANONYMOUS: "Catalogue Abcam" INTERNET ARTICLE, [Online] XP002392221 Retrieved from the Internet: URL:http://www.abcam.com/>

## Description

The invention relates to a method for identifying cell-specific protein combination patterns of cell surfaces of circulating cells of the immune system of a human or animal for the identification of amyotrophic lateral sclerosis (ALS). The invention moreover relates to compositions and kits comprising antibodies and/or ligands which can be employed for this method.

After having sequenced the human genome one of the biggest challenges in human biotechnology is to decipher the networks of the proteins in the cell. To address a larger number of proteins directly in a biological structure several methods have been reported, however the number of simultaneously detectable molecular components in one cell is still too low to analyse the spatial architecture of molecular networks.

Methods for identifying cell-specific protein combination patterns is known from US 6,150,173, DE 197 09 348, EP 0 810 428, DE 100 43 470 and WO 02/21137. The known methods are named MELK (Multi-Epitope-Ligand-Kartographie) and are based on earlier observations indicating that at least 17 different tissue antigens can be labelled by using iterative rounds of protein-tagging steps in one and the same tissue section. The MELK technology has the potential to label hundreds of different molecular components of the cell simultaneously unravelling the modes and rules of molecular network architectures.

Assuming that within, and on the surface, of cells proteins are assembled as networks generating the myriads of cell functionalities, it is to be assumed that these proteins are not stochastically distributed, but timely and spatially highly organized: as in the written language, in which letters, following syntactic rules, are assembled to words, the proteins assembled as networks are likely to be topologically determined in every cell. The cell itself is the corresponding protein pattern formation apparatus, or, protein co-compartmentalization machine. Each protein must be at the right time at the right location at the right concentration in a cell to interact with other proteins. Every protein network is characterized by a unique contextual protein pattern. Hence this indicates that every cellular functionality is likely to be detectable as a specific pattern in situ. The entirety of all protein networks in the cell is termed a toponome (topos = place/position; nomos = law). To identify protein networks in situ an appropriate microscopic "reading" technology must fulfil the following conditions: It must be capable (i) to colocalize a large number of proteins (or other molecular cell components) independently in one and the same cell in one experiment; (ii) to visualize the resulting patterns specifically designating a given cell type, or given cellular function (or dysfunction) in situ; (iii) to "understand" the meaning of these patterns by comparing a given detected pattern with a memory store containing the rules for protein assemblies in the cell (toponome dictionary). MELK provides the principle of colocalising extremely large numbers of different molecular components.

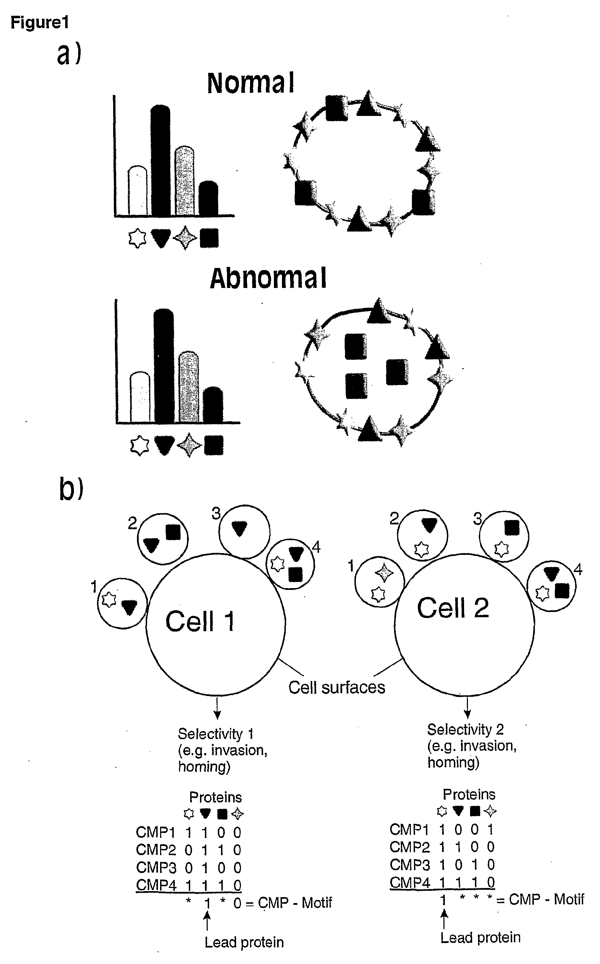

EP 0 810 428 B1 also discloses an apparatus consisting of a multi pipette handling unit, a fluorescence microscope, and a CCD camera, apply large tag-libraries (antibodies, lectins or any other kinds of ligands) on fixed cells or tissues to localize their molecular components. Each tag is conjugated to the same specific dye, for example FITC, and applied sequentially on the sample. Each labelling step is followed by an imaging and a bleaching step, resulting in iterative rounds of labelling, imaging and bleaching (repetitive incubation-imaging-bleaching cycles). The result of these imaging procedures is a protein fingerprint (or molecular component fingerprint) of every subcellular 'data point' in the corresponding sample. The resulting context-dependent protein information, which would be lost in cell homogenates or lists of proteins, supports the view that protein networks are spatially determined functional units in every single cell (different functions = different cellular protein networks = unique cellular protein patterns). The scheme in Fig. 1 a illustrates that single normal and abnormal cells are specifically characterized by the relative order of proteins detected as cellular fingerprints. However, when the cells are destroyed by homogenisation, the quantitative profiles of the corresponding proteins show no difference in this example (Fig. 1a, on the left). The latter profiles would lead to the conclusion that the identified proteins are not relevant for this disease. However, the mapping of the protein pattern networks (Fig. 1a, on the right) leads to the opposite conclusion. Recent experiences support the view that every cell obeys a quasi infinite 'data space' to generate different patterns enciphering different functionalities. The patterns which are actually 'used' by cells in situ (in vivo) are highly restricted and non-random.

MELK supports the search for relevant patterns by providing large data spaces or 'reading frames' to map functional clusters of proteins, i.e. in diseases. From a pure mathematical point of view the gain of information provided by MELK compared to methods extracting proteins from cell homogenates is enormous: given 20 proteins, which are isolated from a cell- or tissue homogenate (classical proteome analysis), and given, that every protein can be detected at 250 different concentrations (0 to 250), the resulting protein profile would contain a maximum number of 25020 different possibilities of protein combinations at different concentrations. However, every cell has the potential to combine the proteins in every single subcellular compartment in a different way. Given, that in every cell out of 1 million cells 2000 subcellular 'data points' can be measured (MELK), the resulting maximum of possible combinations would be 1 Mio. x 2000 x 25020. Hence, the biological information increases exponentially using MELK compared to the classical procedures of protein analysis (walking through large proteome fractions with subcellular resolution). Cellular fingerprints can be traced out surprisingly fast, sometimes within hours or few days (6).

US 2003/175832 A1 discloses methods of monitoring amyotrophic lateral sclerosis (ALS) disease development or progression and monitoring an ALS therapy by determining the level of HLA-DR expression by CD14+ monocytes and/or the percentage of CD16+ cells in the population of CD14+ cells and/or the number of CD14+/CD16+ cells in peripheral blood of an individual with ALS.

BELOV LARISSA ET AL ("Immunophenotyping of leukemias using a cluster of differentiation antibody microarray" CANCER RESEARCH, AMERICAN ASSOCIATION FOR CANCER RESEARCH, BALTIMORE, MD, US, vol. 61, no. 11, 1 June 2001 (2001-06-01), pages 4483-4489) disclose a method for identifying cluster of differentiation antigens on leukocytes for the identification of different stages of leukemias.

WO 01/36939 to which reference is made herewith discloses a mathematical method to define an assembly of proteins exerting a given cell function as protein network motif (formally = combinatorial molecular pattern motif; CMP motif). Thereby every protein is expressed as present or absent related to a certain grey value threshold, determined in each individual MELK data set by an automated approach (present =1, absent =0, 1 bit). Hence, the primary image information consisting in grey value distributions for every protein can be replaced by geometric descriptions of combinatorial binary vectors in x/y/z coordinates (combinatorial molecular patterns, CMPs, Fig. 1 b). Each CMP represents the topologically determined arrangements of proteins on the cell surface. These vectors, translated as geometric objects, can then be visualized as functional images of the whole cell, referred to as toponome maps (not shown). As illustrated in the CMP lists of the two cells (Fig. 1 b), different CMPs may have in common one or more proteins (lead proteins); one or more proteins may never be present (=0); and some proteins may be variably associated with the lead proteins (=*, wild cards). These common features of several CMPs are defined as a CMP motif. As illustrated by comparison of the two cells (Fig. 1 b), these cells use the same proteins to generate different CMP motifs on the cell surface, coding for different selectivities of cell-to-cell and/or cell-to-matrix interactions.

Functional considerations imply that proteins involved in cell-to-cell or cell-to-matrix interactions as well as signal transduction are organized as multimolecular domains on the cell surface. Following the theory of the toponome, the number of existing cell surface proteins contributing to these functions, is likely to be much lower than the number of cell surface selectivities that cells have to establish in a multicellular organism. Hence, a cell must establish the principle of differential combinatorics of these proteins to express the myriads of different cell surface selectivities. Fig. 1b illustrates schematically that two cells use the same proteins to generate different selectivities by different combinations of these proteins.

Beside these advantages and benefits of the known methods and apparatus for identifying cell-specific protein combination patterns of cell surfaces there is still the need for a reliable and relatively uncomplicated identification of the organ-specific chronic inflammatory disease amyotrophic lateral sclerosis (ALS), especially in the subclinical stages of the diseases.

The invention therefore is based on the object of providing an option for the reliable and relatively uncomplicated identification of amyotrophic lateral sclerosis (ALS) and an effective means for the treatment of such disease and especially of subclinical stages of the disease.

This object according to the invention is solved by way of a method, compositions, kits as well as applications with the features of the independent claims.

Further advantageous variations of the invention are defined in the subclaims.

The solution of the object is based on the knowledge that high selectivities of cell-to-cell interaction play an important role in the circulating cells of the immune system. These cells, especially lymphocytes, are dedicated to immuno-surveillance. During these physiological processes, but also during chronic inflammatory diseases, these cells leave the blood circulation and selectively enter organs by a specific interaction of their cell surfaces with the cell surfaces of the organ-specific endothelial cells. The inventive method for identifying cell-specific protein combination patterns of cell surfaces of circulating cells of the immune system of a human or animal for the identification of amyotrophic lateral sclerosis (ALS) comprises therefore the following steps: a) providing a sample of circulating cells of the immune system of a human or animal showing and providing a sample of circulating cells of the same cell type of the immune system of a healthy human or animal, wherein said circulating cells are peripheral blood mononuclear cells (PBMC); b) determining cell-specific protein combination patterns (cell surface toponome motifs) of at least the cell surface proteins HLA-DQ, Fcgamma RIII (CD16) and CD11 on the cell surfaces of said healthy and pathologically modified cells; c) comparing the resulting protein combination patterns of the cell surfaces of said healthy and pathologically modified cells and subtracting the coincident parts of the protein combination patterns of said healthy and pathologically modified cells, thereby determining a cell-specific protein combination patterns of the cell surfaces of said pathologically modified cells resulting therefrom and d) identifying and characterizing the resulting cell-specific protein combination pattern of the cell surfaces of said pathologically modified circulating cells of the immune system in terms of molecules and its spatial arrangement within the cell as disease specific, wherein the disease is amyotrophic lateral sclerosis (ALS) if the cell surface toponome motif of the cell surface proteins HLA-DQ, Fcgamma RIII (CD16) and CD11 in the cell sample is the amyotrophic lateral sclerosis (ALS)-specific cell surface toponome motif, "HLA-DQ *off* / Fcgamma RIII (CD16) *on* / CD11 *off"*. The inventive method demonstrates that there is a highly selective
recognition and invasion of different organs, generally termed homing, which is specifically encoded in the cell surface toponome encompassing the whole homing grammar. This includes pathological invasion processes, for example in organ-specific chronic inflammatory diseases. Consequently a complete cell surface toponome fingerprint of circulating immune cells in the blood will contain all relevant homing and invasion addresses. Moreover in organ-specific diseases, it is possible to provide direct images of abnormal invasion addresses. This implies that the method according to the invention is able to classify the cell surface toponome of circulating immune cells and provides highly predictive data sets for the differentiation of amyotrophic lateral sclerosis (ALS) and to find organ-specific fingerprints already in the subclinical stages of this disease (Fig. 1b).

The spatial arrangement of the cell surface proteins of circulating PBMC cells of the immune system may be captured in an advantageous variation of the invention by iterative rounds of the sequence of the following method steps: a) applying a solution to the cell sample; b) allowing the solution to take effect and removing the solution; c) recording an image prior to or after removing the solution, wherein the solution contains at least one labeled antibody and/or ligand binding specifically to one of the cell surface proteins. This sequence is the core of the already mentioned MELK method which is described in detail in the printed patent specifications US 6,150,173, DE 197 09 348, EP 0 810 428 and to which reference is made herewith. Steps a) to c) may also be repeated with at least one further solution which equally contains at least one labeled antibody and/or ligand binding specifically to one of the further cell surface proteins, and in that possibly subsequent to step b) and/or c) a washing step and/or subsequent to step (c) a bleaching step is performed. The bleaching step may prove necessary or at least advantageous when employing antibodies and/or ligands coupled to the fluorescent dye. Particularly preferred for the identification of the cell-specific protein combination patterns of the cell surfaces is the employment of at least one antibody which is member of the following group:
- antibody which is directed against the cell surface protein CD4, CD8, HLA-DQ, HLA-DR, CD3, CD26, CD38, CD45, Fcgamma RIII (CD16), CD57, CD56, CD7, CD71, CD11, CD36, CD19, or CD2.

Particularly preferred antibodies are fluorescein-labeled or quantum dot labeled antibodies. Besides the fluorescein labelings of course other labelings are possible, too. The named antibodies have proven particularly suitable in a number of tests.

In a particularly preferred variation of the invention the MELK robot technology is used for the detection of the disease-specific protein combination pattern of the cell surfaces of said pathologically modified circulating cells of the immune system.

The above mentioned object is also solved by providing compositions or kits, which comprise at least one antibody and/or at least one ligand, wherein the antibodies and/or ligands bind the cell surface protein HLA-DQ, Fcgamma RIII (CD16), and CD11 specifically and are coupled each labelings, in particular with different labelings. If different labelings are used, the individual antibodies and/or ligands and
thus also the cell surface protein bound through them can be differentiated in the case of a simultaneously performed labeling. By employing the composition according to the invention the time required for the identification of the CMP motif is considerably shortened and the performance of the above method facilitated. In a kit the individual antibodies and/or ligands are contained in different receptacles, but are still made available to the user simultaneously. When using such a kit it is not necessary that the labelings of the cell surface proteins are performed simultaneously, so that the antibodies and/or ligands may also be coupled with identical labelings. In a preferred variation of the invention at least one of the labelings is a fluorescent dye, in particular phycoerythrin or fluorescein, or a quantum dot.

The composition or kit may comprise at least one further antibody and/or ligand, wherein the at least one further antibody and/or ligand binds the cell surface protein CD4, CD8, HLA-DR, CD3, CD26, CD38, CD45, CD57, CD56, CD7, CD71, CD36, CD19, or CD2 specifically and is coupled with a labeling.

The described compositions or kits are suitable for being employed in the identification of ALS and especially of subclinical stages of the disease as part of a laboratory test.

According to the invention also a biochip is provided, wherein on one surface of the chip ligands and/or antibodies binding specifically to the ALS-specific protein combination pattern "HLA-DQ off / Fcgamma RIII (CD16) on / CD11 off" of the cell surface of pathologically modified circulating cells of the immune system are coupled in such a way that their bondability to the specific protein combination pattern of the cell surface is sustained.

The above-mentioned object according to the invention is also solved by the use of the described inventive composition or kit or biochip for preparing a means of the diagnosis of ALS.

The above-mentioned object according to the invention is also solved by the use of the described inventive composition or kit or biochip for preparing a means of the diagnosis of ALS. This is because it could be shown that Fcgamma RIII (CD16) plays a central role during the different stages of ALS, so that this process is prevented by inhibiting the Fcgamma RIII (CD16) by aid of a corresponding human antibody.

The above-mentioned object according to the invention is also solved by the use of the amyotrophic lateral sclerosis (ALS)-specific cell surface toponome motif, "HLA-DQ off / Fcgamma RIII (CD16) on / CD11 off" of the cell surfaces of pathologically modified peripheral blood mononuclear cells for the development of antibodies directed against this pattern for preparing a remedy for the treatment of amyotrophic lateral sclerosis (ALS).

Additional features and advantages of the invention derive from the following description of an example supporting the inventive concept.

### Example: Amyotrophic Lateral Sclerosis (ALS)

The inventive method was applied to the analysis of the cell surface toponome of peripheral blood mononuclear cells (PBMC) using an antibody library against 18 cell surface proteins, most of which belong to the class of adhesion molecules. The antibody library used was directed against the following cell surface proteins (following the cluster of differentiation-antigen-nomenclature):
CD4, CD8, HLA-DQ, HLA-DR, CD3, CD26, CD38, CD45, Fcgamma RIII (CD16), CD57, CD56, CD7, CD71, CD11, CD36, CD19, and CD2.

Additional features and advantages of the invention derive from the following description of an example supporting the inventive concept.

### Example: Amyotrophic Lateral Sclerosis (ALS)

The inventive method was applied to the analysis of the cell surface toponome of peripheral blood mononuclear cells (PBMC) using an antibody library against 18 cell surface proteins, most of which belong to the class of adhesion molecules. The antibody library used was directed against the following cell surface proteins (following the cluster of differentiation-antigen-nomenclature):
CD4, CD8, HLA-DQ, HLA-DR, CD3, CD26, CD38, CD45, Fcgamma RIII (CD16), CD57, CD56, CD7, CD71, CD11, CD36, CD19, and CD2.

Briefly, the PBMC were isolated form the blood by a Ficoll gradient, then diluted in PBS, placed on cover slips, air dried, fixed for 10 sec in acetone, and then snap frozen in isopentane pre-cooled in liquid nitrogen. Immediately before use for the toponome measurements according to the invention the cells on the cover slips were fixed in acetone at minus 20° C, air dried and then rehydrated in PBS. The cells were then subjected to measurements as described in the inventive methods described above.

Seven individuals suffering from the sporadic form of ALS and eight healthy control individuals were investigated. Since the methods according to the invention are preferably only performed in the laboratory, the sample-taking preferably is not part of the method according to the invention.

As a major result a statistically significant cell surface toponome motif was found in the ALS patients that was either not observed or with only a very low frequency in normal controls.

The motif carries the following characteristic features:

| | |
|---|---|
| CD4, | * |
| CD8, | * |
| HLA-DQ, | 0 |
| HLA-DR, | * |
| CD3, | * |
| CD26, | * |
| CD38, | * |
| CD45, | * |
| Fcgamma RIII (CD16), | 1 |
| CD57, | * |
| CD56, | * |
| CD7, | * |
| CD71, | * |
| CD11, | 0 |
| CD36, | * |
| CD19, | * |
| CD2. | * |

Together this motif shows that in the blood of ALS patients there is an accumulation of PBMC which are characterized by constant presence of the Fcgamma RIII receptor (1), which, by a rule, is anti-colocalized for CD11 (0) and HLA-DQ (0), while the other cell surface proteins indicated as wild cards (*) are variably associated with Fcgamma RIII, e.g. may be present (1)(*on*) or absent (0)(*off*) in may different combinatorial variations in individual single PBMC (characteristics of a motif by definition). Together 246 variations (cell types) of this motif were found. PMBC carrying this cell surface toponome motif are statistically significant in ALS (p=0.019, T test).

The sporadic form of ALS is characterized by cell death of motor neurons in the CNS (pyramidal tract), and also involves the second peripheral motor neuron, whose cell bodies are located in the anterior horn of the spinal cord. While the cause of this neuronal cell death is unknown, it is well known that ALS is accompanied by presence of some CD8 positive immune cells in the spinal cord pyramidal tract, which represent the myelinated axons of the first central motor neuron projecting to the anterior horn cells of the spinal cord to link the central with the peripheral motor neuron.

Upon routine current classic immunocytochemical techniques these CD8 positive cells are not conspicious to directly alter the motor neuron axons (McGeer PLM & McGeer EG : Muscle Nerve 26, 459 - 470, 2002). Therefore the current scientific opinion is that these cells invade the spinal cord as a secondary response to the degeneration of the first motor neuron, rather than playing a primary part in motor neuron death mechanisms.

In order to investigate this issue by using the inventive method, post mortem spinal cord tissue was analyzed using the same above described antibody library.

The following post mortem tissues provided were studied:

| **case** | **statement** | **sex** | **age** | **neurology** | **course (years)** | **cause of death** |
|---|---|---|---|---|---|---|
| 1 | ALS | m | 67 | bulbar | 1 | respiratory insufficiency |
| 2 | ALS | f | 60 | bulbar | 2 | respiratory insufficiency |
| 3 | ALS | m | 73 | atrophic | 2 | respiratory insufficiency |
| 4 | control | f | 39 | - | | aneurysm rupture |
| 5 | control | f | 75 | - | | cardiac insufficiency |

As a result of these studies the following new features of ALS were found:
In the lumen of post capillary venules which are present in the pyramidal tract of the spinal cord, single PBMC were found during their penetration of the capillary endothelium (a process that is commonly defined as homing).

These same cells expressed precisely the abnormal cell surface toponome phenotype as the PMBC found to be specifically occurring in the blood of ALS patients: The same toponome motif, with Fcgamma RIII as a lead protein (see above).

Several different stages of this homing process were found showing that the Fcgamma RIII is internalized at the rear polarized site in these cells while the front site protrudes across the endothelium. After this penetration process the cells are located between the myelinated neurons projecting thin long cell extensions penetrating the myelin sheath of morphologically intact single motor neurons forming a hook-like anchor within the motor axon. In that stage of penetration the cells do not show any expression of the Fcgamma RIII but a strong co-expression of CD3, CD8 and HLADR at the very tip indenting the axon.

By analyzing the normal control spinal cords, homing phenomena of single PBMC were also seen, but by striking contrast, these cells never expressed the Fcgamma RIII, and instead of CD8 co-expressed CD4, while the other associated molecules were different from those seen as a motif in ALS (absence of the specific variations of protein combinations present in ALS). Another striking difference of these normal homing phenomena was that after penetration of these cells to the motor neurons, these cells never formed cell projections, but instead reside strictly between the myelin sheaths without attacking the motor neuron myelin.

There are several new conclusions based on the inventive method that now can be drawn concerning the cellular pathogenesis of ALS.
1. The peripheral ALS-Toponome motif on blood PBMC is obviously a predictive combinatorial molecular phenotype of ALS, because PBMC with precisely the same cell surface toponome motif home to the spinal cord pyramidal tract. In contrast to current knowledge it is now clear for the first time that ALS pathogenesis relies on specific abnormal immune cells detectable in the blood.
2. The fact that these invasive PBMC axotomize motor neuron axons by penetrating the myelin sheaths strongly suggests that these cells actively and selectively damage the myelinated axons of the first motor neuron. While the cause of this specific attack is not known at present, this result provides direct evidence that the detected abnormal PBMC type plays an active role in the motor neuron damage. This result relies on the high "functional" (multi-molecular combinatorial) resolution of the inventive method thereby unravelling a more active role of invasive PBMC than previously thought.

These data strongly support the new concept of the generative grammar of the cell surface toponome to produce a large number of highly selective invasion addresses by differential combination of cell surface proteins generated by PBMC.

Most importantly, by applying the method according to the invention to PBMC it is now possible to progressively decipher the combinatorial invasion/homing code of PBMC in the blood in many diseases, such as chronic inflammatory diseases, e.g. cellular autoimmune diseases (chronic hepatitis, diseases of the rheumatoid class, thyroiditis, Multiple sclerosis) and in cancer, in which also selective PBMC invasion/homing into the tumour sites is observed. Hence analyses according to the inventive method of blood samples in these diseases will provide specific cellular toponome fingerprints predictive for both the diseased organ, and most likely the type of disease. The above described antibody library is claimed to be of central importance for such detection and prediction procedures based on the present inventive analyses of the blood.

## Claims

1. A method for identifying a cell-specific cell surface toponome motif of circulating cells of the immune system of a human or animal for the identification of amyotrophic lateral sclerosis (ALS) comprising the following steps:
a) providing a sample of pathologically modified circulating cells of the immune system of a human or animal showing an organ-specific chronic inflammatory disease and providing a sample of circulating cells of the same cell type of the immune system of a healthy human or animal, wherein said circulating cells are peripheral blood mononuclear cells (PBMC);
b) determining cell-specific cell surface toponomes of at least the cell surface proteins HLA-DQ, Fcgamma RIII (CD16) and CD11 in the cell samples of said healthy and pathologically modified cells;
c) comparing the resulting cell surface toponomes of said healthy and pathologically modified cells and subtracting the coincident parts of the cell surface toponomes of said healthy and pathologically modified cells, thereby determining a cell-specific cell surface toponome motif of said pathologically modified cells resulting therefrom; and
d) identifying and characterizing the resulting cell-specific cell surface toponome motif of said pathologically modified circulating cells of the immune system in terms of molecules and its spatial arrangement within the cell as disease specific, wherein the disease is amyotrophic lateral sclerosis (ALS) if the cell surface toponome motif of the cell surface proteins HLA-DQ, Fcgamma RIII (CD16) and CD11 in the cell sample is the amyotrophic lateral sclerosis (ALS)-specific cell surface toponome motif, "HLA-DQ *off*/ Fcgamma RIII (CD16) *on* / CD11 *off"*.

2. Method according to claim 1, **characterized in that** the cells are lymphocytes.

3. Method according to claim 1 or 2, **characterized in that** the resulting cell-specific cell surface toponome motif of said pathologically modified circulating cells of the immune system contains homing and invasion addresses of organ-specific endothelial cells.

4. Method according to one of the previous claims, **characterized in that** the spatial arrangement of the cell surface proteins of circulating cells of the immune system is captured by iterative rounds of the sequence of the following method steps:
a) applying a solution to the cell sample;
b) allowing the solution to take effect and removing the solution;
c) recording an image prior to or after removing the solution;
wherein the solution contains at least one labeled antibody and/or ligand binding specifically to one of the cell surface proteins.

5. Method according to claim 4, **characterized in that** steps (a) to (c) are repeated with at least one further solution which also contains at least one labeled antibody and/or ligand specifically binding to one of the further cell surface proteins, and **in that** possibly subsequent to step (b) and/or (c) a washing step and/or subsequent to step (c) a bleaching step is performed.

6. Method according to claims 4 or 5, **characterized in that** at least one antibody is employed which is member of the following group:
- antibody which is directed against the cell surface protein CD4, CD8, HLA-DQ, HLA-DR, CD3, CD26, CD38, CD45, Fcgamma RIII (CD16), CD57, CD56, CD7, CD71, CD11, CD36, CD19, or CD2.

7. Method according to claim 6, **characterized in that** the antibodies are fluorescein-labeled or quantum dot labeled antibodies or labeled by any other fluorescent marker.

8. Method according to one of the previous claims, **characterized in that** the Multi-Epitope-Ligand-Kartographie (MELK) robot technology is used for the detection of the disease-specific cell surface toponome motif of said pathologically modified circulating cells of the immune system.

9. Composition or kit for use in the method of claims 1 to 8, comprising three antibodies and/or ligands, wherein the antibodies and/or ligands bind the cell surface proteins HLA-DQ, Fcgamma RIII (CD16), and CD11 specifically and are coupled each with labelings, in particular with different labelings.

10. Composition or kit according to claim 9, comprising at least one further antibody and/or ligand, wherein the at least one further antibody and/or ligand binds the cell surface protein CD4, CD8, HLA-DR, CD3, CD26, CD38, CD45, CD57, CD56, CD7, CD71, CD36, CD19, or CD2 specifically and is coupled with a labeling.

11. Composition or kit according to claim 9 or 10, **characterized in that** at least one of the labelings is a fluorescent dye, in particular phycoerythrin or fluorescein, or a quantum dot.

12. Biochip for use in the method of claims 1, 2 or 3, wherein on one surface of the chip ligands and/or antibodies binding specifically to the amyotrophic lateral sclerosis (ALS)-specific cell surface toponome motif, "HLA-DQ *off* / Fcgamma RIII (CD16) *on* / CD11 *off"* of the cell surface of pathologically modified circulating cells of the immune system are coupled in such a way that their bondability to the specific cell surface toponome motif of the cell surface is sustained.

13. Composition or kit or biochip according to one of the claims 9 to 12 for use in the *in vitro* diagnosis of ALS.

14. Use of pathologically modified peripheral blood mononuclear cells for the development of antibodies directed against the amyotrophic lateral sclerosis (ALS)-specific cell surface toponome motif, "HLA-DQ *off* / Fcgamma RIII (CD16) *on* / CD11 *off"* in the circulating cells for preparing a remedy for the treatment of amyotrophic lateral sclerosis (ALS).

15. A method for the identification of amyotrophic lateral sclerosis (ALS) comprising the steps:
- providing a sample of peripheral blood mononuclear cells (PBMC) of a human or animal;
- determining cell-specific cell surface toponomes of at least the cell surface proteins HLA-DQ, Fcgamma RIII (CD16) and CD11 in the sample, and
- identifying and characterizing the resulting cell-specific cell surface toponome motif of said sample in terms of molecules and its spatial arrangement within the cell as disease specific, wherein
the disease is amyotrophic lateral sclerosis (ALS) if the cell surface toponome motif of the cell surface proteins HLA-DQ, Fcgamma RIII (CD16) and CD11 in the cell sample is the amyotrophic lateral sclerosis (ALS)-specific cell surface toponome motif, "HLA-DQ *off* / Fcgamma RIII (CD16) *on* / CD11 *off"*.

## Patentansprüche

1. Verfahren zum Identifizieren eines zellenspezifischen Zellenoberflächentoponommusters von zirkulierenden Zellen des Immunsystems eines Menschen oder Tiers für die Identifikation von amyotropher Lateralsklerose (ALS) mit den folgenden Schritten:
a) Bereitstellen einer Probe von pathologisch modifizierten zirkulierenden Zellen des Immunsystems eines Menschen oder Tiers, der bzw. das eine organspezifische chronische Entzündungserkrankung zeigt, und Vorsehen einer Probe von zirkulierenden Zellen desselben Zellentyps des Immunsystems eines gesunden Menschen oder Tiers, wobei die zirkulierenden Zellen mononukleäre Zellen des peripheren Bluts (PBMC) sind;
b) Bestimmen von zellenspezifischen Zellenoberflächentoponomen von zumindest den Zellenoberflächenproteinen HLA-DQ, Fcgamma RIII (CD16) und CD11 in den Zellenproben der gesunden und pathologisch modifizierten Zellen;
c) Vergleichen der resultierenden Zellenoberflächentoponome der gesunden und pathologisch modifizierten Zellen und Subtrahieren der übereinstimmenden Teile der Zellenoberflächentoponome der gesunden und pathologisch modifizierten Zellen, wodurch ein zellenspezifisches Zellenoberflächentoponommuster der pathologisch modifizierten Zellen, das sich daraus ergibt, bestimmt wird; und
d) Identifizieren und Charakterisieren des resultierenden zellenspezifischen Zellenoberflächentoponommusters der pathologisch modifizierten zirkulierenden Zellen des Immunsystems hinsichtlich Molekülen und seiner räumlichen Anordnung innerhalb der Zelle als für die Erkrankung spezifisch, wobei die Erkrankung amyotrophe Lateralsklerose (ALS) ist, wenn das Zellenoberflächentoponommuster der Zellenoberflächenproteine HLA-DQ, Fcgamma RIII (CD16) und CD11 in der Zellenprobe das für die amyotrophe Lateralsklerose (ALS) spezifische Zellenoberflächentoponommuster "HLA-DQ *off* / Fcgamma RIII (CD16) *on* / CD11 *off"* ist*.*

2. Verfahren nach Anspruch 1, **dadurch gekennzeichnet, dass** die Zellen Lymphozyten sind.

3. Verfahren nach Anspruch 1 oder 2, **dadurch gekennzeichnet, dass** das resultierende zellenspezifische Zellenoberflächentoponommuster der pathologisch modifizierten zirkulierenden Zellen des Immunsystems Homing- und Invasions-Adressen von organspezifischen Endothelzellen enthält.

4. Verfahren nach einem der vorangehenden Ansprüche, **dadurch gekennzeichnet, dass** die räumliche Anordnung der Zellenoberflächenproteine von zirkulierenden Zellen des Immunsystems durch iterative Runden der Sequenz der folgenden Verfahrensschritte erfasst wird:
a) Aufbringen einer Lösung auf die Zellenprobe;
b) Ermöglichen, dass die Lösung einwirkt, und Entfernen der Lösung;
c) Aufzeichnen eines Bildes vor oder nach dem Entfernen der Lösung;
wobei die Lösung mindestens einen markierten Antikörper und/oder Liganden enthält, der spezifisch an eines der Zellenoberflächenproteine bindet.

5. Verfahren nach Anspruch 4, **dadurch gekennzeichnet, dass** die Schritte (a) bis (c) mit mindestens einer weiteren Lösung wiederholt werden, die auch mindestens einen markierten Antikörper und/oder Liganden enthält, der spezifisch an eines der weiteren Zellenoberflächenproteine bindet, und dass möglicherweise im Anschluss an Schritt (b) und/oder (c) ein Waschschritt und/oder im Anschluss an Schritt (c) ein Bleichschritt durchgeführt wird.

6. Verfahren nach den Ansprüchen 4 oder 5, **dadurch gekennzeichnet, dass** mindestens ein Antikörper verwendet wird, der ein Mitglied der folgenden Gruppe ist:
- ein Antikörper, der gegen das Zellenoberflächenprotein CD4, CD8, HLA-DQ, HLA-DR, CD3, CD26, CD38, CD45, Fcgamma RIII (CD16), CD57, CD56, CD7, CD71, CD11, CD36, CD19 oder CD2 gerichtet ist.

7. Verfahren nach Anspruch 6, **dadurch gekennzeichnet, dass** die Antikörper mit Fluoreszein markierte oder mit Quantenpunkt markierte Antikörper sind oder mit irgendeinem anderen Fluoreszenzmarker markiert sind.

8. Verfahren nach einem der vorangehenden Ansprüche, **dadurch gekennzeichnet, dass** die Multi-Epitop-Ligand-Kartographie- (MELK) Robotertechnologie für die Detektion des für die Erkrankung spezifischen Zellenoberflächentoponommusters der pathologisch modifizierten zirkulierenden Zellen des Immunsystems verwendet wird.

9. Zusammensetzung oder Ausrüstung für die Verwendung im Verfahren nach den Ansprüchen 1 bis 8 mit drei Antikörpern und/oder Liganden, wobei die Antikörper und/oder Liganden die Zellenoberflächenproteine HLA-DQ, Fcgamma RIII (CD16) und CD11 spezifisch binden und jeweils mit Markierungen, insbesondere mit verschiedenen Markierungen, gekoppelt sind.

10. Zusammensetzung oder Ausrüstung nach Anspruch 9 mit mindestens einem weiteren Antikörper und/oder Liganden, wobei der mindestens eine weitere Antikörper und/oder Ligand das Zellenoberflächenprotein CD4, CD8, HLA-DR, CD3, CD26, CD38, CD45, CD57, CD56, CD7, CD71, CD36, CD19 oder CD2 spezifisch bindet und mit einer Markierung gekoppelt ist.

11. Zusammensetzung oder Ausrüstung nach Anspruch 9 oder 10, **dadurch gekennzeichnet, dass** mindestens eine der Markierungen ein Fluoreszenzfarbstoff, insbesondere Phykoerythrin oder Fluoreszein, oder ein Quantenpunkt ist.

12. Biochip für die Verwendung im Verfahren der Ansprüche 1, 2 oder 3, wobei auf einer Oberfläche des Chips Liganden und/oder Antikörper, die spezifisch an das für die amyotrophe Lateralsklerose (ALS) spezifische Zellenoberflächentoponommuster "HLA-DQ *off*/ Fcgamma RIII (CD16) *on* / CD11 *off"* der Zellenoberfläche von pathologisch modifizierten zirkulierenden Zellen des Immunsystems binden, in einer solchen Weise gekoppelt sind, dass ihre Bindungsfähigkeit an das spezifische Zellenoberflächentoponommuster der Zellenoberfläche aufrechterhalten wird.

13. Zusammensetzung oder Ausrüstung oder Biochip nach einem der Ansprüche 9 bis 12 zur Verwendung bei der In-vitro-Diagnose von ALS.

14. Verwendung von pathologisch modifizierten mononukleären Zellen des peripheren Bluts für die Entwicklung von Antikörpern, die gegen das für die amyotrophe Lateralsklerose (ALS) spezifische Zellenoberflächentoponommuster "HLA-DQ *off*/ Fcgamma RIII (CD16) *on* / CD11 *off"* in den zirkulierenden Zellen gerichtet sind, zur Herstellung eines Heilmittels für die Behandlung von amyotropher Lateralsklerose (ALS).

15. Verfahren für die Identifikation von amyotropher Lateralsklerose (ALS) mit den Schritten:
- Bereitstellen einer Probe von mononukleären Zellen des peripheren Bluts (PBMC) eines Menschen oder Tiers;
- Bestimmen von zellenspezifischen Zellenoberflächentoponomen von zumindest den Zellenoberflächenproteinen HLA-DQ, Fcgamma RIII (CD16) und CD11 in der Probe, und
- Identifizieren und Charakterisieren des resultierenden zellenspezifischen Zellenoberflächentoponommusters der Probe hinsichtlich Molekülen und seiner räumlichen Anordnung innerhalb der Zelle als für die Erkrankung spezifisch, wobei
die Erkrankung amyotrophe Lateralsklerose (ALS) ist, wenn das Zellenoberflächentoponommuster der Zellenoberflächenproteine HLA-DQ, Fcgamma RIII (CD16) und CD11 in der Zellenprobe das für amyotrophe Lateralsklerose (ALS) spezifische Zellenoberflächentoponommuster "HLA-DQ *off* / Fcgamma RIII (CD16) *on* / CD11 *off"* ist.

## Revendications

1. Procédé d'identification d'un modèle toponymique de surface de cellule spécifique d'une cellule de cellules circulantes du système immunitaire d'un être humain ou d'un animal pour l'identification de la sclérose latérale amyotrophique (SLA), comprenant les étapes suivantes, consistant à :
a) fournir un échantillon de cellules circulantes pathologiquement modifiées du système immunitaire d'un être humain ou d'un animal montrant une maladie inflammatoire chronique spécifique d'un organe et fournir un échantillon de cellules circulantes du même type de cellule du système immunitaire d'un être humain ou d'un animal sain, dans lequel lesdites cellules circulantes sont des cellules mononuclées de sang périphérique (CMSP) ;
b) déterminer des toponomes de surface de cellule spécifiques d'une cellule d'au moins les protéines de surface de cellule HLA-DQ, Fc gamma RIII (CD16) et CD11 dans les échantillons de cellules desdites cellules saines et pathologiquement modifiées ;
c) comparer les toponomes de surface de cellule résultants desdites cellules saines et pathologiquement modifiées et soustraire les parties coïncidentielles des toponomes de surface de cellule desdites cellules saines et pathologiquement modifiées, déterminant par ce moyen un modèle toponymique de surface de cellule spécifique d'une cellule desdites cellules pathologiquement modifiées qui en résultent et
d) identifier et caractériser le modèle toponymique résultant de surface de cellule spécifique d'une cellule desdites cellules circulantes pathologiquement modifiées du système immunitaire en termes de molécules et de son agencement spatial à l'intérieur de la cellule, comme étant spécifique d'une maladie, dans lequel la maladie est la sclérose latérale amyotrophique (SLA) si le modèle toponymique de surface de cellule des protéines de surface de cellule HLA-DQ, Fc gamma RIII (CD16) et CD11 dans l'échantillon de cellule est le modèle toponymique « HLA-DQ *absent* / Fc gamma RIII (CD16) *présent* / CD11 *absent* » de surface de cellule spécifique de la sclérose latérale amyotrophique (SLA).

2. Procédé selon la revendication 1, **caractérisé en ce que** les de cellules sont des lymphocytes.

3. Procédé selon la revendication 1 ou 2, **caractérisé en ce que** le modèle toponymique résultant de surface de cellule spécifique d'une cellule desdites cellules circulantes pathologiquement modifiées du système immunitaire contient des adresses de domiciliation et d'invasion de cellules endothéliales spécifiques d'un organe.

4. Procédé selon l'une des revendications précédentes, **caractérisées en ce que** l'agencement spatial des protéines de surface de cellule de cellules circulantes du système immunitaire est cerné par des passages itératifs de la séquence des étapes suivantes du procédé consistant à :
a) appliquer une solution à l'échantillon de cellule ;
b) permettre à la solution de prendre effet et supprimer la solution ;
c) enregistrer une image avant de supprimer ou après avoir supprimé la solution ;
dans lequel la solution contient au moins un anticorps et / ou un ligand marqué qui se lie spécifiquement à l'une des protéines de surface de cellule.

5. Procédé selon la revendication 4, **caractérisé en ce que** les étapes (a) à (c) sont répétées avec au moins une solution supplémentaire qui contient aussi au moins un anticorps et / ou un ligand marqué qui se lie spécifiquement à l'une des protéines de surface de cellule supplémentaires et **en ce que**, éventuellement postérieurement à l'étape (b) et (c), une étape de lavage et / ou, postérieurement à l'étape (c), une étape de blanchiment est exécutée.

6. Procédé selon les revendications 4 ou 5, **caractérisé en ce qu'**au moins un anticorps est employé, qui fait partie du groupe suivant :
- anticorps qui est dirigé contre la protéine de surface de cellule CD4, CD8, HLA-DQ, HLA-DR, CD3, CD26, CD38, CD45, Fc gamma RIII (CD16), CD57, CD56, CD7, CD71, CD1 1, CD36, CD19 ou CD2.

7. Procédé selon la revendication 6, **caractérisé en ce que** les anticorps sont marqués à la fluorescéine ou sont des anticorps marqués aux boîtes quantiques ou sont marqués par tout autre marqueur fluorescent.

8. Procédé selon l'une des revendications précédentes, **caractérisé en ce que** la technique robotique de la cartographie de ligand multi-épitopique (CLME) est utilisée pour la détection du modèle toponymique de surface de cellule spécifique d'une maladie desdites cellules circulantes pathologiquement modifiées du système immunitaire.

9. Composition ou ensemble à usage dans le procédé des revendications 1 à 8, comprenant trois anticorps et / ou ligands, dans lequel les anticorps et / ou ligands se lient aux protéines de surface de cellule HLA-DQ, Fc gamma RIII (CD16) et CD11 spécifiquement et sont associés chacun à des marquages, en particulier à des marquages différents.

10. Composition ou ensemble selon la revendication 9, comprenant au moins un anticorps et / ou ligand supplémentaire, dans lequel le au moins un anticorps et / ou ligand supplémentaire se lie à la protéine de surface de cellule CD4, CD8, HLA-DR, CD3, CD26, CD38, CD45, CD57, CD56, CD7, CD71, CD36, CD19 ou CD2 spécifiquement et est associé à un marquage.

11. Composition ou ensemble selon la revendication 9 ou 10, **caractérisé en ce qu'**au moins l'un des marquages est un colorant fluorescent, en particulier de la phycoérythrine ou de la fluorescéine ou une boîte quantique.

12. Puce biologique pour usage dans le procédé des revendications 1, 2 ou 3, dans laquelle, sur une surface de la puce, des ligands et / ou anticorps se liant spécifiquement au modèle toponymique « HLA-DQ *absent* / Fc gamma RIII (CD16) *présent* / CD11 *absent* de surface de cellule spécifique de la sclérose latérale amyotrophique (SLA), de la surface de cellule de cellules circulantes pathologiquement modifiées du système immunitaire sont associés d'une manière telle que leur aptitude à la liaison par rapport au modèle toponymique de surface de cellule spécifique de la surface de cellule est maintenue.

13. Composition ou ensemble ou puce biologique selon l'une des revendications 9 à 12, à usage dans le diagnostic *in vitro* de la SLA.

14. Utilisation de cellules mononuclées du sang périphérique pathologiquement modifiées pour le développement d'anticorps dirigés contre le modèle toponymique « HLA-DQ *absent* / Fc gamma RIII (CD16) *présent* / CD11 *absent* de surface de cellule spécifique de la sclérose latérale amyotrophique (SLA) dans les cellules circulantes, pour préparer un remède pour le traitement de la sclérose latérale amyotrophique (SLA).

15. Procédé d'identification de la sclérose latérale amyotrophique (SLA), comprenant les étapes consistant à :
- fournir un échantillon de cellules mononuclées de sang périphérique (CMSP) d'un être humain ou d'un animal ;
- déterminer des toponomes de surface de cellule spécifiques d'une cellule d'au moins les protéines de surface de cellule HLA-DQ, Fc gamma RIII (CD16) et CD11 dans l'échantillon et
- identifier et caractériser le modèle toponymique résultant de surface de cellule spécifique d'une cellule dudit échantillon en termes de molécules et son agencement spatial à l'intérieur de la cellule comme étant spécifique d'une maladie, dans lequel
la maladie est la sclérose latérale amyotrophique (SLA) si le modèle toponymique de surface de cellule des protéines de surface de cellule HLA-DQ, Fc gamma RIII (CD16) et CD11 dans l'échantillon de cellule est le modèle toponymique « HLA-DQ *absent* / Fc gamma RIII (CD16) *présent* / CD11 *absent* de surface de cellule spécifique de la sclérose latérale amyotrophique (SLA).
